# EUROPEAN PATENT APPLICATION

(11) **EP 4 585 590 A1**
(43) Date of publication of application: **16.07.2025**
(21) Application number: 24186109.5
(22) Date of filing: 02.07.2024
(51) Int. Cl.: C07D 295/088

(54) **METHOD FOR RECOVERING HYDROXYETHYL PIPERAZINE**

(71) Applicant: BASF SE, 67056 Ludwigshafen am Rhein (DE)
(72) Inventor: JOEDECKE, Michael, 67056 Ludwigshafen am Rhein (DE)
(74) Representative: Meissner Bolte Partnerschaft mbB

(57) **Abstract**

A method for recovering hydroxyethyl piperazine from an aqueous piperazine ethoxylation mixture comprises a) introducing the piperazine ethoxylation mixture as a side feed into a distillation column fluidly connected to a still while continuously separating the mixture into a distillate containing aqueous piperazine and bottoms containing hydroxyethyl piperazine and bishydroxyethyl piperazine, and collecting the bottoms in the still; and b) when the still has reached a predetermined filling level, discontinuing introducing the piperazine ethoxylation mixture into the distillation column, and raising the temperature of the bottoms in the still and/or lowering the column pressure to obtain a hydroxyethyl piperazine-containing overhead stream and a bishydroxyethyl piperazine-containing residue remaining in the still. During separation of unreacted piperazine, problems caused by solidification of piperazine leading to clogging of distillation column or distillation column condenser are avoided.

## Description

The present invention relates to a method for recovering hydroxyethyl piperazine from an aqueous piperazine ethoxylation mixture.

Hydroxyethyl piperazine (1-(2-hydroxyethyl)piperazine) is an intermediate of the alkylated piperazines family. It serves mainly as an absorbent for the sweetening of gas streams, building block for the production of active pharmaceutical ingredients and of buffering agents used in cell culture. Hydroxyethyl piperazine has the following structure:

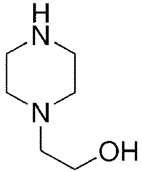

Hydroxyethyl piperazine may be formed by an ethoxylation reaction of piperazine, i.e. the reaction of piperazine with ethylene oxide. The ethoxylation reaction of piperazine produces both hydroxyethyl piperazine and bishydroxyethyl piperazine as an unwanted side product. The resultant mixture contains water and unreacted piperazine in addition to hydroxyethyl piperazine and bishydroxyethyl piperazine. Additional processing steps, e.g., by distillation are required to purify the product mixture to form a desired product essentially consisting of hydroxyethyl piperazine. The process may be modified to favor the production of hydroxyethyl piperazine over bishydroxyethyl piperazine. Low molar ratios of ethylene oxide to piperazine allow for the production of hydroxyethyl piperazine to dominate, however, may result in significant amounts of unreacted piperazine. Separation of the unreacted piperazine at a commercial process scale is difficult as piperazine solidifies at room temperature and may clog a distillation column or distillation column condenser.

As such, there is a need for a process for recovering hydroxyethyl piperazine from an aqueous piperazine ethoxylation mixture, that addresses the issues associated with piperazine solidification.

EP 3873886 A relates to a continuous process for the production of hydroxyethyl piperazine that includes feeding neat piperazine, recycled piperazine, and ethylene oxide to a reactor to form crude hydroxyethyl piperazine. The process further includes continuously feeding the crude hydroxyethyl piperazine from the reactor to a distillation system that includes at least one distillation column: The distillation system produces at least a recycled piperazine stream and a hydroxyethyl piperazine stream; the recycled piperazine stream includes the recycled piperazine that is fed to the reactor to form the crude hydroxyethyl piperazine.

The invention relates to a method for recovering hydroxyethyl piperazine from an aqueous piperazine ethoxylation mixture, comprising:
a) introducing the piperazine ethoxylation mixture as a side feed into a distillation column fluidly connected to a still while continuously separating the mixture into a distillate containing aqueous piperazine and bottoms containing hydroxyethyl piperazine and bishydroxyethyl piperazine, and collecting the bottoms in the still;
b) when the still has reached a predetermined filling level, discontinuing introducing the piperazine ethoxylation mixture into the distillation column, and raising the temperature of the bottoms in the still and/or lowering the column pressure to obtain a hydroxyethyl piperazine-containing overhead stream and a bishydroxyethyl piperazine-containing residue remaining in the still.

In an embodiment, the method additionally comprises, after step b):
c) introducing water into the still to liquefy the bishydroxyethyl piperazine-containing residue, and removing the liquefied residue from the still.

Generally, the aqueous piperazine ethoxylation mixture comprises 25 to 30 wt.-% of piperazine, 25 to 30 wt.-% of water, 25 to 30 wt.-% of hydroxyethyl piperazine and up to 15 wt.-%, typically about 10 wt.-%, of bishydroxyethyl piperazine.

The inventive method utilizes a distillation column fluidly connected to a still (still kettle or stillpot). "Fluidly connected" is intended to mean that rising vapors and trickling liquid can exchange between the distillation column and the still. The still is equipped with an externally heated reboiler. The column has an an externally cooled overhead condenser. In the condenser, latent heat is removed as the vapors are condensed and partially returned as reflux which cascades down the column. The column may be any conventional distillation column such as a plate column, e.g., a perforated plate column, a bubble-cap column, a Kittel tray column, a uniflux tray column, and a ripple tray column; or a packed column.

Batch separation processes by means of a still with an attached distillation column are known and described in the literature, for example in "Thermal Separation Process" by K. Sattler, edition 1988, VCH Verlagsgesellschaft, Weinheim, pages 161-162.

The inventive method comprises two steps. Step a) is carried out as a semi-batch distillation. In step a), the feed of the piperazine ethoxylation mixture is supplied at such a rate that aqueous piperazine as the low-boiling component is concurrently distilled off.

When piperazine is distilled together with water as an extracting solvent, no condition arises in the column in which the temperature is below the melting point of piperazine and the concentration of piperazine is higher than its solubility in water. Therefore, piperazine does not solidify and does not clog the distillation column.

Conveniently, step a) is carried out at a column pressure of 200 mbar and a distillation head temperature of 60 to 70 °C.

The aqueous piperazine that is distilled off in step a) is a valuable product that may be recycled into the piperazine ethoxylation reaction or may be used elsewhere, e.g., in chemical synthesis or as a constituent of adsorbent solutions for the sweetening of gaseous streams such as natural gas. If desired, the obtained aqueous piperazine solution may be subjected to separation to obtain anhydrous piperazine.

Bottoms containing hydroxyethyl piperazine and bishydroxyethyl piperazine are collected in the still. No bottoms are withdrawn from the still during step a).

When the still has reached a predetermined filling level, e.g., when the still is full, introduction of the piperazine ethoxylation mixture into the distillation column is discontinued. Then, step b) is carried out as batch distillation. To this end, the temperature of the bottoms in the still is raised and/or the column pressure is lowered, preferably the column pressure is lowered, to obtain a hydroxyethyl piperazine-containing overhead stream and a bishydroxyethyl piperazine-containing residue remaining in the still.

Conveniently, step b) is carried out at a starting column pressure of 100 mbar which is stepwise reduced to 20 mbar, and a bottoms temperature of 160 to 180 °C.

Discharge of the bishydroxyethyl piperazine-containing residue remaining in the still is not a trivial task due to its high melting point of about 160 °C. Withdrawal of molten bishydroxyethyl piperazine would require trace heating of all pipings and pumps which would increase both investment and operational costs. Even then, clogging problems may prevail.

Hence, in an embodiment, it is suggested to introduce water into the still to liquefy the bishydroxyethyl piperazine-containing residue, and removing the liquefied residue from the still. To this end, the vacuum in the column may be relieved and heating of the bottoms discontinued. The water which is introduced in the still cools the bottom residue and reduces its melting point. Suitably, about 60 to 70 wt.-% of water, relative to the weight of the bishydroxyethyl piperazine-containing residue, are introduced into the still. This reduces the melting point of the residue and the liquefied residue becomes pumpable without clogging. The liquefied residue may be discharged from the still with any suitable pumping means such as conventional fluid pumps.

The ethoxylation reaction which yields the aqueous piperazine ethoxylation mixture may be carried out as follows.

The ethoxylation is typically performed batchwise, for example in stirred autoclaves or loop reactors, at a reaction temperature in the range of from 80 to 200 °C, and at a reaction pressure in the range of from 2 to 3 bara. An aqueous piperazine solution is charged to the autoclave or reactor. Then, the desired amount of ethylene oxide is injected.

Preferably, the aqueous piperazine solution has a piperazine concentration in the range of from 40 to 90 wt.-%, more preferably 55 to 75 wt.-%, based on the weight of the piperazine solution. At least a part of the piperazine solution may be the distillate of step a) as described above.

In the ethoxylation reaction, a catalyst may be used. Suitable catalysts are basic compounds, for example alkali metal alkoxides, or Lewis acids.

For safety reasons owing to the flammability of ethylene oxide, the process is typically performed under inert atmosphere, such as nitrogen or another inert gas. The partial pressure of the ethylene oxide reactant is preferably limited by dilution with the inert gas, to a gas phase concentration of 50% or less. This ensures safety against explosion-like polyaddition of the ethylene oxide.

The tank reactor is preferably inertized, i.e., purged with an inert gas to displace any oxygen and other gases that may be detrimental to the addition reaction. Preferably, the tank reactor is purged with an inert gas and evacuated several times. Nitrogen, argon, methane or carbon dioxide may be used as inert gases. For reasons of its better availability and thus of its low price, nitrogen is the preferred inert gas.

Generally, thorough mixing of the contents of the reactor should be ensured in all the reaction phases. To this end, an agitator, an external circulation system or a combination thereof may be adopted.

The ethylene oxide addition reaction is highly exothermic. The temperature within the reactor is kept at the desired level or adjusted to the desired level by cooling. Cooling is generally carried out via the reactor wall and/or by means of heat exchanger surfaces arranged internally in the reactor and/or externally in the pumped circulation, e.g. on cooling coils, cooling cartridges, plate heat exchangers, tube bundle heat exchangers or mixer heat exchangers.

Metering of the ethylene oxide into the reactor may occur through nozzles, in particular nozzles in the bottom of the tank reactor or through a distributor ring immersed in the liquid. The introduction of stirring energy into the liquid phase causes mixing, during which a small amount of the ethylene oxide is dissolved in the liquid phase; the remaining amount forms a gas phase above the liquid with the gas used for inertization.

Once the addition of ethylene oxide is finished, he obtained aqueous piperazine ethoxylation mixture is subjected to step a) of the inventive method for recovering hydroxyethyl piperazine as described in detail above.

The invention is further illustrated by the accompanying drawing.

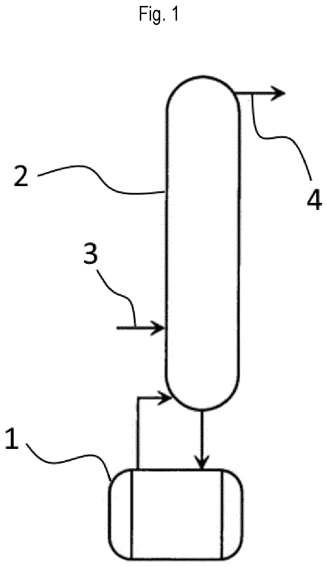

Referring to Fig. 1, a distillation apparatus comprises a still kettle 1 and a distillation column 2 fluidly connected to the still kettle 1. A piperazine ethoxylation mixture is fed to the distillation column 2 via side feed 3. Temperature and pressure are set appropriately to separate the mixture into a distillate containing aqueous piperazine which is withdrawn overhead via line 4, and bottoms containing hydroxyethyl piperazine and bishydroxyethyl piperazine, which are collected in the still kettle 1.

When the still kettle 1 has reached a predetermined filling level, introduction of the piperazine ethoxylation mixture into the distillation column 2 is discontinued. The temperature of the bottoms in the still is raised and/or the distillation column 2 pressure is lowered to obtain a hydroxyethyl piperazine-containing overhead stream via line 4 and a bishydroxyethyl piperazine-containing residue remaining in the still kettle 1.

Once hydroxyethyl piperazine is essentially distilled off via line 4, the vacuum in the column is relieved and heating of the bottoms is discontinued. Then, water is introduced into the still via side feed 3 to liquefy the bishydroxyethyl piperazine-containing residue in the still kettle 1. Finally, the liquefied residue is removed from the still kettle 1 via a piping (not shown) using a pump (not shown). Then, feeding of a piperazine ethoxylation mixture to the distillation column 2 and separation of the mixture into a distillate containing aqueous piperazine and bottoms is resumed.

List of reference signs
- 1: still kettle
- 2: distillation column
- 3: side feed
- 4: line

## Claims

1. A method for recovering hydroxyethyl piperazine from an aqueous piperazine ethoxylation mixture, comprising:
a) introducing the piperazine ethoxylation mixture as a side feed into a distillation column fluidly connected to a still while continuously separating the mixture into a distillate containing aqueous piperazine and bottoms containing hydroxyethyl piperazine and bishydroxyethyl piperazine, and collecting the bottoms in the still;
b) when the still has reached a predetermined filling level, discontinuing introducing the piperazine ethoxylation mixture into the distillation column, and raising the temperature of the bottoms in the still and/or lowering the column pressure to obtain a hydroxyethyl piperazine-containing overhead stream and a bishydroxyethyl piperazine-containing residue remaining in the still.

2. The method according to claim 1, additionally comprising, after step b):
c) introducing water into the still to liquefy the bishydroxyethyl piperazine-containing residue, and removing the liquefied residue from the still.

3. The method according to claim 1 or 2, wherein the aqueous piperazine ethoxylation mixture comprises 25 to 30 wt.-% of piperazine, 25 to 30 wt.-% of water, 25 to 30 wt.-% of hydroxyethyl piperazine and about 10 wt.-% of bishydroxyethyl piperazine.

4. The method according to any one of the preceding claims, wherein step a) is carried out at a column pressure of 200 mbar and a distillation head temperature of 60 to 70 °C.

5. The method according to any one of the preceding claims, wherein step b) is carried out at a starting column pressure of 100 mbar which is stepwise reduced to 20 mbar, and a bottoms temperature of 160 to 180 °C.

6. The method according to claim 2, wherein in step c) about 60 to 70 wt.-% of water, relative to the weight of the bishydroxyethyl piperazine-containing residue, are introduced into the still.

7. The method according to any one of the preceding claims, wherein prior to step a), a batchwise ethoxylation reaction comprises
- charging an aqueous piperazine solution into a stirred autoclave or a loop reactor,
- injecting a predetermined amount of ethylene oxide into the stirred autoclave or the loop reactor to obtain a reaction mixture, and
- reacting the reaction mixture at a predetermined reaction temperature to obtain an aqueous piperazine ethoxylation mixture to be subjected to step a).

8. The method according to claim 7, wherein the reaction temperature is in the range of from 80 to 200 °C.
